# EUROPÄISCHE PATENTSCHRIFT

(11) **EP 2 732 839 B1**
(45) Veröffentlichungstag und Bekanntmachung des Hinweises auf die Patenterteilung: **12.05.2021**
(21) Anmeldenummer: 13005324.2
(22) Anmeldetag: 12.11.2013
(51) Int. Cl.: A61M 16/06, F16F 1/377

(54) **Vorrichtung zur Positionierung eines Patienten Interfaces**
Device for positioning a patient interface
Dispositif de positionnement d'une interface de patient

(30) Priorität: 15.11.2012 DE 102012022355
(43) Veröffentlichungstag der Anmeldung: 21.05.2014
(73) Patentinhaber: Löwenstein Medical Technology S.A., 2557 Luxembourg (LU)
(72) Erfinder: Feldhahn, Karl-Andreas, 22761 Hamburg (DE); Frerichs, Arnold, 21614 Buxtehude (DE); Bechtel, Martin, 21423 Winsen / Luhe (DE)
(74) Vertreter: Marx, Thomas

(56) Entgegenhaltungen:
- EP-A1- 0 438 760
- EP-A1- 0 637 699
- EP-A2- 0 320 608
- EP-A2- 2 005 985
- EP-A2- 2 005 986
- WO-A1-02/063179
- WO-A1-2012/020359
- DE-A1- 10 106 597
- DE-U1-202013 100 755
- US-A- 5 280 890
- US-A1- 2007 215 161
- US-A1- 2011 094 516
- US-A1- 2012 012 113

## Beschreibung

### Gebiet der Erfindung

Die Erfindung betrifft eine Vorrichtung zur Positionierung eines Patienten Interfaces am Kopf des Anwenders.

### Stand der Technik

Patienten Interfaces dienen dazu, vom Beatmungsgerät bereitgestelltes Atemgas an den Patienten abzugeben. Patienten Interfaces können in unterschiedlichen Ausführungsformen beispielsweise Nasal- oder Vollgesichtsmasken realisiert sein. Das Patienten Interface ist typischerweise über einen Atemgasschlauch mit dem Beatmungsgerät verbunden und wird am Kopf des Anwenders fixiert.

Da das Patienten Interface stunden-, bzw. nächtelang vom Patienten getragen werden muss, sind hohe Anforderungen an den Tragekomfort gestellt.

Neben der genauen Passform des Patienten Interfaces ist die Befestigung und Fixierung am Kopf des Anwenders wichtig um unangenehme Druckstellen und Undichtigkeiten bzw. Leckagen zu vermeiden.

### Kritik am Stand der Technik

Zur Gewährleistung einer sicheren Positionierung des Patienten Interfaces im Bereich des Gesichtes eines Patienten, sowie der Verringerung der Kräfte die auf das Gesicht wirken, werden Patienten Interfaces mit Stirnstützen benutzt. Derartige Stirnstützen sind für den Patienten aber oft zu kompliziert und meist nur aufwendig einstellbar.

Die bisher auf dem Markt verfügbaren Stirnstützen sind oftmals mit komplizierten Verstellmechanismen versehen und teilweise nicht selbsterklärend, sowie bei EP2005985.

Der Anwender muss sich nach der Demontage - die zur Reinigung seiner Maske notwendig ist - die Rastposition der Stirnstütze merken und immer wieder erneut einstellen.

### Erfindung

Diese Erfindung betrifft Verstellvorrichtung für ein Patienten Interface welche eine genaue, einfache Positionierung im Gesicht eines Patienten gewährleistet und die Erfindung wird nur durch Ansprüche 1-12 begrenzt.

Aufgabe der vorliegenden Erfindung ist es, eine Verstellvorrichtung für ein Patienten Interface anzugeben welche eine einfache Positionierung des Patienten Interfaces ermöglicht und der Tragekomfort des Patienten Interfaces verbessert.

Beschrieben ist eine Verstellvorrichtung für ein Patienten Interface, welches mittels einer Kopfbänderung am Kopf des Patienten fixiert wird, mit einem Stützkörper und zumindest einem Federelement wobei das Patienten Interface zumindest eine Bänderungs-Aufnahme aufweist und die Anzugskraft der Kopfbänderung über die Bänderungs-Aufnahme auf das Federelement übertragen wird, sodass das Federelement komprimiert wird.

Die Verstellvorrichtung ist auch dadurch gekennzeichnet, dass der Stützkörper zumindest teilweise in eine horizontale Aufnahme des Maskenkörpers eingreift.

Die Verstellvorrichtung ist auch dadurch gekennzeichnet, dass eine Aufnahme für das Federelement im Bereich der Aufnahme und/oder im Stützkörper angeordnet ist.

Die Verstellvorrichtung ist auch dadurch gekennzeichnet, dass das Federelement im montierten Zustand teilweise in die Aufnahme und teilweise in die Führung eintaucht und damit über die gesamte Länge überdeckt ist.

Die Verstellvorrichtung ist auch dadurch gekennzeichnet, dass die Führung außen Nasen aufweist, die auf Stegen federnd angeordnet sind und mit den Nasen bei der Montage der Stützkörper in den Aufnahmebereich der horizontalen Aufnahme greift und dort verrastet.

Die Verstellvorrichtung ist auch dadurch gekennzeichnet, dass sich zur verdreh-sicheren Montage außen an der Federaufnahme Führungselemente befinden, die in entsprechende innenliegende Führungsnuten der Aufnahme greifen.

Die Verstellvorrichtung ist auch dadurch gekennzeichnet, dass die zylindrische Aufnahme am Ende eine Anlagefläche für das Federelement aufweist und der Federweg durch diese Anlagefläche und die Länge der Aufnahmebereiche begrenzt ist.

Die Verstellvorrichtung ist auch dadurch gekennzeichnet, dass das Federelement zumindest teilweise in die Öffnung der Stirnstützenaufnahme eingeführt ist und die Fläche im Hinterschnitt liegt und so in der Öffnung der Stirnstützenaufnahme gehalten wird.

Die Verstellvorrichtung ist auch dadurch gekennzeichnet, dass die zylindrische Führung des Stützkörpers über das Federelement geführt ist und mit den Nasen in dem Aufnahmebereich der Stirnstützenaufnahme verrastet.

Die Verstellvorrichtung ist auch dadurch gekennzeichnet, dass das Federelement an der Anlagefläche in dem Stützkörper anliegt und der Federweg durch die Anlagefläche und die gewählte Länge der Aufnahmebereiche begrenzt wird.

Die Verstellvorrichtung ist auch dadurch gekennzeichnet, dass das Federelement X-förmig angeordnete Streben aufweist.

Die Verstellvorrichtung ist auch dadurch gekennzeichnet, dass die Federelementkonstante des Federelementes im Bereich zwischen 0,1 - 2,0 N/mm und liegt.

Die Verstellvorrichtung ist auch dadurch gekennzeichnet, dass die Federelementkonstante im Bereich zwischen 0,1 - 1,0 N/mm liegt.

Die Verstellvorrichtung ist auch dadurch gekennzeichnet, dass Kennlinie des Federelementes annähernd linear ist.

Die Verstellvorrichtung ist auch dadurch gekennzeichnet, dass die Kennlinie des Federelementes durch Verwendung unterschiedlicher Elastomermaterialien mehrstufig ist.

Die Verstellvorrichtung ist auch dadurch gekennzeichnet, dass Kennlinie des Federelementes durch Verwendung unterschiedlicher Geometrien oder geometrischer Aussparungen (entlang der Achse oder quer zur Achse) innerhalb des Federelementes mehrstufig ist.

Die Verstellvorrichtung ist auch dadurch gekennzeichnet, dass das Federelement einen Federweg im Bereich 5 mm bis 30 mm bereitstellt.

Die Verstellvorrichtung ist auch dadurch gekennzeichnet, dass das Federelement einen Federweg im Bereich 7 mm bis 20 mm bereitstellt.

Die Verstellvorrichtung ist auch dadurch gekennzeichnet, dass Federelement in einem Teilbereich nicht von den Führungselementen umschlossen ist und so der Reinigung zugänglich ist.

Die Verstellvorrichtung ist auch dadurch gekennzeichnet, dass Federelement eine Achse aufweist in deren Richtung die Feder gestaucht wird.

Die Verstellvorrichtung ist auch dadurch gekennzeichnet, dass die Achse der Feder im Wesentlichen senkrecht zur Stirn des Anwenders orientiert ist.

Die Verstellvorrichtung ist auch dadurch gekennzeichnet, dass das Federelement senkrecht zur Achse einen ersten Teilbereich mit einer ersten Eigenschaft (beispielsweise Geometrie oder Elastizität) und senkrecht zum ersten Teilbereich und zur Achse einen zweiten Teilbereich mit einer zweiten Eigenschaft aufweist.

Die Verstellvorrichtung ist auch dadurch gekennzeichnet, dass die erste Eigenschaft ungleich der zweiten Eigenschaft ist.

Die Verstellvorrichtung ist bevorzugt auch dadurch gekennzeichnet, dass das Federelement einen ersten und einen zweiten Teilbereich aufweist, die einander gegenüberliegen wobei der erste Teilbereich eine höhere Steifigkeit aufweist als der zweite Teilbereich.

Die Verstellvorrichtung ist auch dadurch gekennzeichnet, dass ein Teilbereich durch geometrische Strukturen (wie beispielsweise Verrippung, offene oder geschlossene Kontur) eine höhere Steifigkeit aufweist.

Die Verstellvorrichtung ist auch dadurch gekennzeichnet, dass ein Teilbereich durch unterschiedliche Materialien (welche beispielsweise im 2K-Verfahren verbunden sind und beispielsweise unterschiedliche Shorehärten im Bereich A 20 - 80 aufweisen) eine erhöhte Steifigkeit aufweist.

Die Verstellvorrichtung ist auch dadurch gekennzeichnet, dass ein Teilbereich durch unterschiedliche Wandstärken eine höhere Steifigkeit aufweist.

Beschrieben ist eine eine Verstellvorrichtung für ein Patienten Interface, welches mittels einer Kopfbänderung am Kopf des Patienten fixiert wird, mit einem Stützkörper und zumindest einem Federelement dadurch gekennzeichnet, das Federelement eine Achse aufweist, in deren Richtung das Federelement gestaucht wird, und wobei das Federelement senkrecht zur Achse eine ersten Teilbereich mit einer ersten Eigenschaft und ortsverschieden zum ersten Teilbereich einen zweiten Teilbereich mit einer zweiten Eigenschaft aufweist.

Beschrieben ist eine Verstellvorrichtung für ein Patienten Interface welches mittels einer Kopfbänderung am Kopf des Patienten fixiert wird, mit einem Stützkörper und zumindest einem Federelement dadurch gekennzeichnet, dass die Federelementkonstante des Federelementes im Bereich zwischen 0,1 - 2,0 N/mm und liegt.

Die Erfindung bezieht sich auch auf eine Verstellvorrichtung für ein Patienten Interface welches mittels einer Kopfbänderung am Kopf des Patienten fixiert wird, mit einem Stützkörper und zumindest einem Federelement dadurch gekennzeichnet, dass das Federelement einen Federweg im Bereich 5 mm bis 30 mm bereitstellt.

Beschrieben ist ein Federelement für ein Patienten Interface mit einer Achse dadurch gekennzeichnet, dass das Federelement senkrecht zur Achse eine ersten Teilbereich mit einer ersten Eigenschaft und ortsverschieden zum ersten Teilbereich einen zweiten Teilbereich mit einer zweiten Eigenschaft aufweist.

Beschrieben ist eine Verstellvorrichtung für ein Patienten Interface, welches mittels einer Kopfbänderung am Kopf des Patienten fixiert wird, vorgeschlagen mit einem Stützkörper und einer Aufnahme für den Stützkörper wobei ein Federelement zwischen Stützkörper und Aufnahme angeordnet ist und Bänderungs-Aufnahmen die Anzugskraft der Kopfbänderung zumindest teilweise auf das Federelement übertragen.

Die Vorrichtung ist auch dadurch gekennzeichnet, dass das Federelement eine stufenlose Verstellung ermöglicht.

Die Vorrichtung ist auch dadurch gekennzeichnet, dass das Federelement einen Federweg im Bereich 7 mm bis 17 mm bereitstellt.

Die Vorrichtung ist auch dadurch gekennzeichnet, dass das Federelement einen Federweg im Bereich 7 mm bis 20 mm bereitstellt.

Die Vorrichtung ist auch dadurch gekennzeichnet, dass der Stützkörper zumindest teilweise in die horizontale Aufnahme des Maskenkörpers eingreift.

Die Vorrichtung ist auch dadurch gekennzeichnet, dass der Stützkörper zumindest teilweise über die horizontale Aufnahme des Maskenkörpers greift.

Die Vorrichtung ist auch dadurch gekennzeichnet, dass die Rückstellkraft des Federelementes und die Rückstellkraft des Stirnauflagepolsters so ausgelegt sind, dass sich beim Anziehen der Bänderung zuerst das Stirnauflagepolster der Form der Anwenderstirn anpasst und erst dann das Federelement komprimiert wird.

Die Vorrichtung ist auch dadurch gekennzeichnet, dass das Federelement im Bereich Aufnahme fixiert wird.

Die Vorrichtung ist auch dadurch gekennzeichnet, dass das Federelement im Bereich der zylindrischen Führung im Stützkörper fixiert wird.

Die Vorrichtung ist auch dadurch gekennzeichnet, dass das Federelement im montierten Zustand teilweise in die Aufnahme und teilweise in die zylindrische Führung eintaucht und damit vollständig überdeckt ist.

Die Vorrichtung ist auch dadurch gekennzeichnet, dass das Federelement im montierten Zustand teilweise in die Aufnahme und teilweise in die zylindrische Führung eintaucht und damit teilweise überdeckt ist.

Die Vorrichtung ist auch dadurch gekennzeichnet, dass die zylindrischen Führung zumindest teilweise in die Aufnahme eintaucht und dort lösbar fixiert ist.

Die Vorrichtung ist auch dadurch gekennzeichnet, dass die zylindrische Führung außen Nasen aufweist, die auf Stegen federnd angeordnet sind und mit den Nasen bei der Montage der Stützkörper in die Aufnahmebereiche der zylindrischen horizontalen Aufnahme greifen und dort leicht verrasten.

Die Vorrichtung ist auch dadurch gekennzeichnet, dass sich zur verdrehsicheren Montage außen an der zylindrischen Federaufnahme Führungselemente befinden, die in entsprechende innenliegende Führungsnuten der Aufnahme greifen und dort geführt werden.

Die Vorrichtung ist auch dadurch gekennzeichnet, dass die zylindrische Aufnahme am Ende eine Anlagefläche für das Federelement aufweist und der Federweg durch diese Anlagefläche und die Länge der Aufnahmebereiche begrenzt ist.

Die Vorrichtung ist auch dadurch gekennzeichnet, dass das Federelement zumindest teilweise in die Öffnung der Stirnstützenaufnahme eingeführt ist und die Fläche im Hinterschnitt liegt und so in der Öffnung der Stirnstützenaufnahme gehalten wird.

Die Vorrichtung ist auch dadurch gekennzeichnet, dass die zylindrische Führung der Stützkörper über das Federelement geführt und mit den Nasen in den Aufnahmebereichen der Stirnstützenaufnahme verrastet.

Die Vorrichtung ist auch dadurch gekennzeichnet, dass das Federelement an der Anlagefläche in dem Stützkörper anliegt und der Federweg durch die Anlagefläche und die gewählte Länge der Aufnahmebereiche begrenzt wird.

Die Vorrichtung ist auch dadurch gekennzeichnet, dass die Elastomer-Feder ihre Funktion als Druckfeder durch die X-förmige Anordnung von Streben und die Auswahl des Materials erhält.

Die Vorrichtung ist auch dadurch gekennzeichnet, dass das Federelementkonstante im Bereich zwischen 0,1 - 2,0 N/mm liegt. Bevorzugt im Bereich 0,1 - 1,0 N/mm, besonders bevorzugt im Bereich 0,1 - 0,8 N/mm oder auch im Bereich 0,1 - 0,5 N/mm.

Die Federkennlinie beschreibt die Abhängigkeit der Federkraft F vom Federweg. Ein Federelement mit einer linearen Federkennlinie hat eine Federkonstante. Denkbar sind auch progressive oder degressive Kennlinien. Die Federkonstante entspricht der Steifigkeit des Federelementes, dividiert durch die Länge. Die Steifigkeit ist der Widerstand eines Federleementes gegen elastische Verformung durch eine Kraft. Bei Verwendung zweier unterschiedlicher Materialien oder Geometrien können zwei unterschiedliche Federkonstanten in einem Federelement realisiert werden, mit jeweils unterschiedlicher Kennlinie und/oder Steifigkeit. Bei elastomeren Federelementen können solche Eigenschaften im 2k Verfahren hergestellt werden.

Je nach Anzugskraft der Bänderung der Kopfbänderung bzw. der Kopfhaube und der Form des Anwendergesichts- bzw. der Anwenderstirn drückt sich das Federelement innerhalb der Stirnstütze mehr oder weniger fest zusammen und drückt das Stirnpolster auf die Stirn des Anwenders und bietet so immer den patientengerechten Abstand des Patienten Interfaces.

Die folgenden Figuren zeigen Ausführungsbeispiele eines Patienten Interfaces mit einem Stützkörper der einleitenden Art.
- Fig.1:: Patienten Interface
- Fig.1a:: Patienten Interface mit Bänderung
- Fig.2:: Explosionsdarstellung der Stirnstützenverstellung, Variante 1
- Fig.3:: Stirnauflage
- Fig.4:: Maskenkörper
- Fig.5:: Elastomer-Feder
- Fig.6:: Explosionsdarstellung der Stirnstützenverstellung, Variante 2
- Fig.7:: Stirnauflage
- Fig.8:: Maskenkörper
- Fig.9:: Elastomer-Feder
- Fig.10:: Explosionsdarstellung der Stirnstützenverstellung, Variante 3
- Fig.11: Stirnauflage
- Fig.12: Maskenkörper
- Fig.13: elastomeres Federelement
- Fig.14: Federelement, Ansicht seitlich
- Fig.14a: Federelement, Ansicht von oben
- Fig.15a,b: Verstellweg Federelement

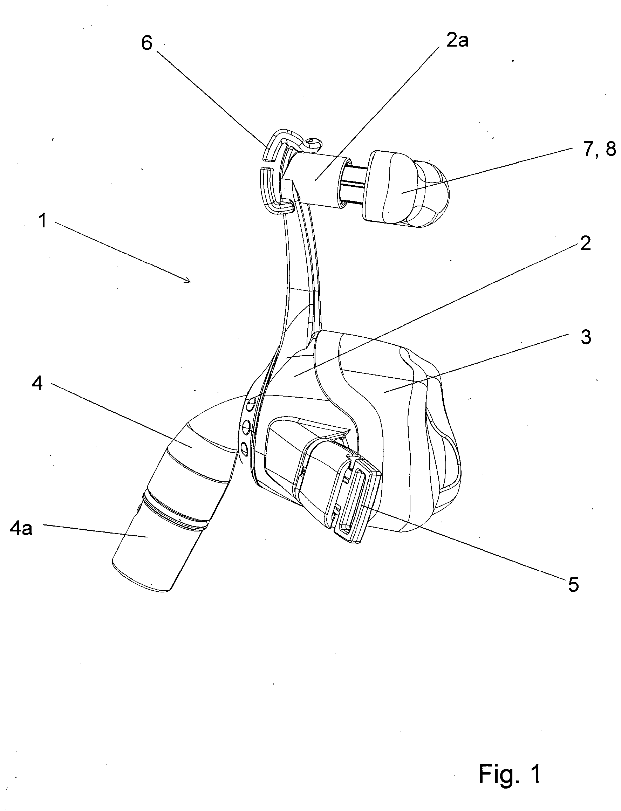

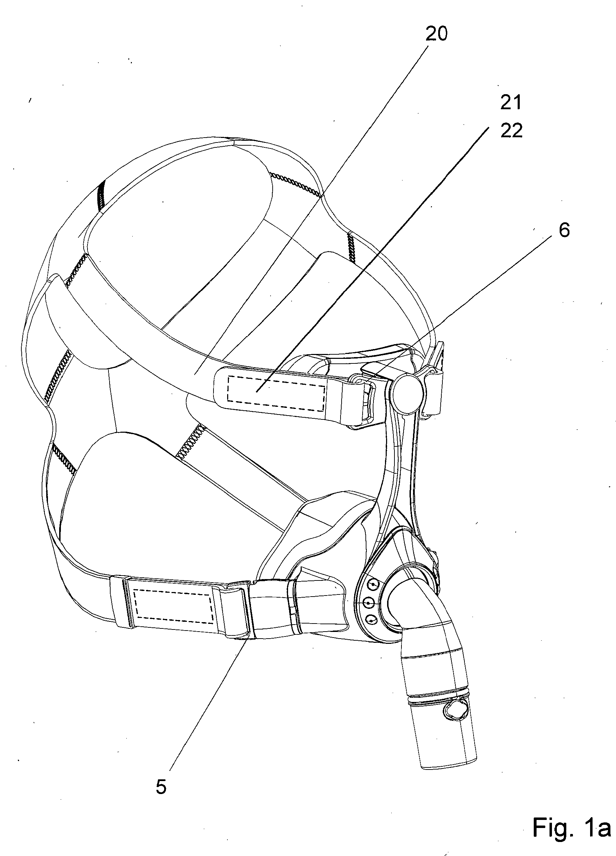

Die Figuren 1 und 1a zeigen ein Patienten Interface (1). Das Patienten Interface (PI) weist einen Maskenkörper (2) auf, an dem über ein Kugel-Gelenk (4) und eine Drehhülse (4a) der nicht dargestellte Atemgasschlauch mit dem PI verbunden wird. Der Maskenkörper (2) weist als Abdichtung relativ zum Gesicht des Patienten ein Dichtelement. (3) in Form eines Maskenwulstes mit einer Lippendichtung auf. Die Fixierung im Bereich des Kopfes eines Patienten kann über eine Kopfhaube oder Kopfbänderung (20) erfolgen. Die Bänderungsenden (22) der Kopfhaube bzw. Kopfbänderung werden zum Einen über Aufnahmevorrichtungen (5) im Wangenbereich und zum Anderen über Aufnahmen (6) im Stirnbereich lösbar an dem PI befestigt. Dazu werden die Bänderungsenden (22) durch die Aufnahmen (6) geführt und über eine Klettverbindung (21) an der Bänderung fixiert. Die Aufnahmen (6) weisen einen Schlitz zum Einführen der Bänderung auf. Zur Abstützung des Patienten Interfaces (1) im Bereich der Stirn des Patienten dient ein Stützkörper (7) mit einem Stirnauflagepolster (8). Der Stützkörper (7) greift in die horizontale Aufnahme (2a) des Maskenkörpers (2).

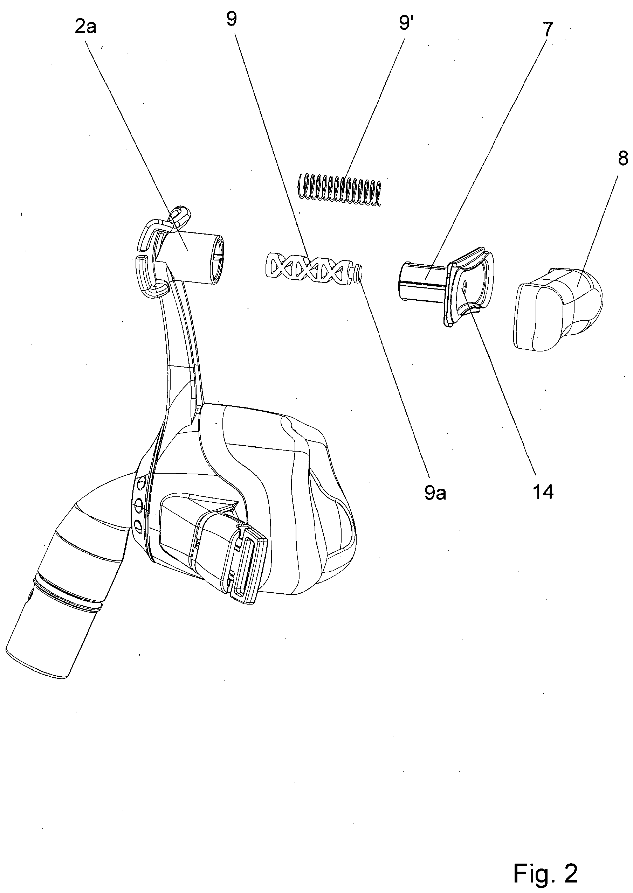

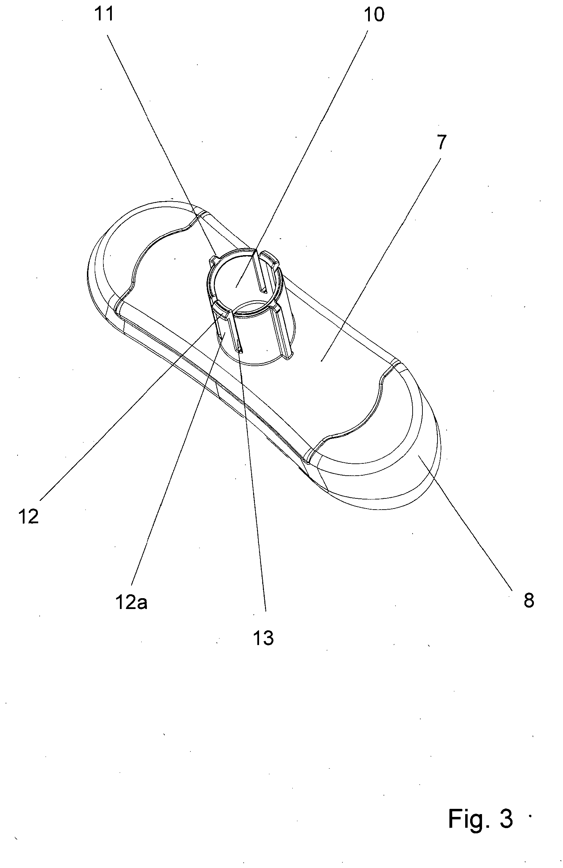

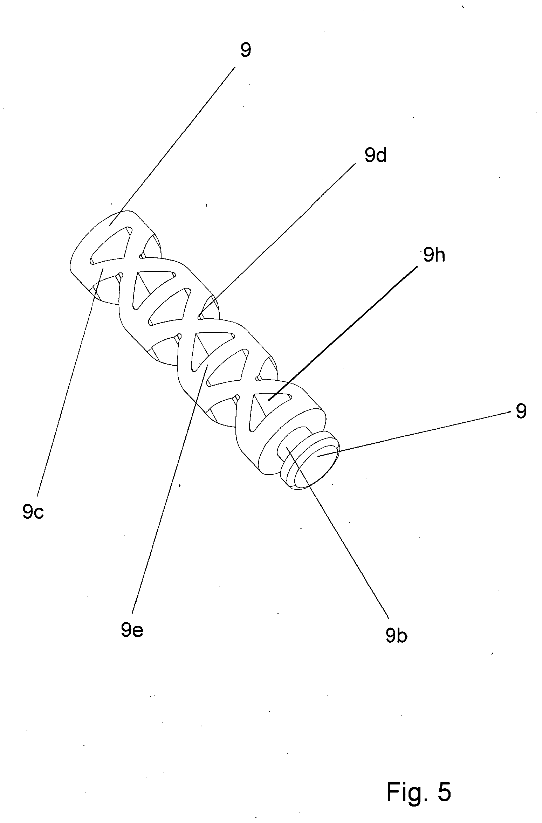

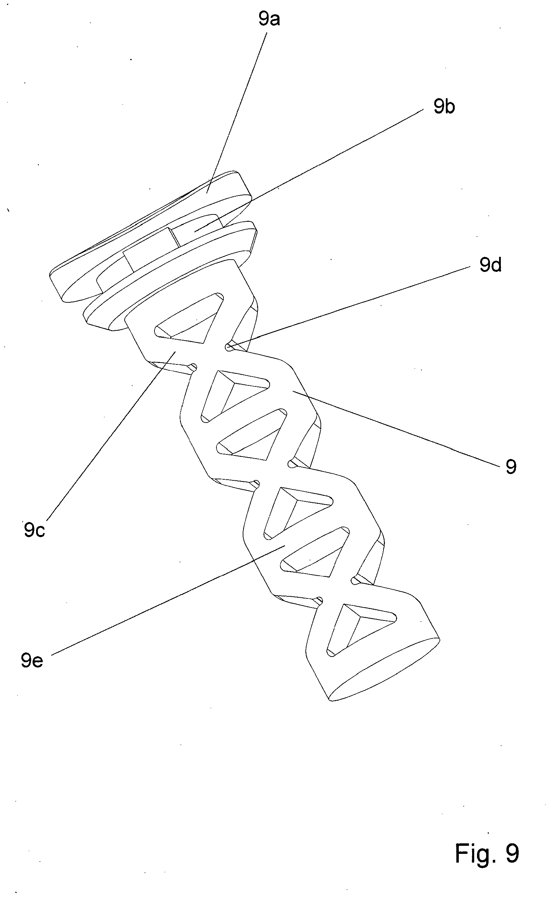

Figur 2 zeigt, dass der Stützkörper (7) der Stirnstütze patientenseitig ein Stirnpolster (8) aufweist, welches aus einem hautfreundlichen Silikon hergestellt ist und auch mit einer Gelfüllung ausgestattet sein kann. Zur Aufnahme des Federelementes (9, 9') dient eine zylindrische Führung (10) (siehe Fig. 3). Das Federelement (9) wird in die Aufnahme (10) gesteckt, in dessen Boden eine zusätzliche Öffnung (14) angeordnet ist, in der das Federelement durch eine pilzköpfige Verdickung (9a) und einen umlaufenden Hinterschnitt (9b) gehalten wird, sodass das Federelement (9) bei der Demontage bzw. zur täglichen Reinigung der Maske nicht verloren gehen kann. Für eine Druckfeder (9') kann eine vergleichbare Sicherung vorgesehen sein.
Eine automatische Verstellung / Einstellung des Stützkörpers (7) erfolgt mittels einem innenliegenden Federelement (9, 9') in dem Stützkörper. Das Federelement kann in allen Ausführungsbeispielen auch außenliegend, beispielsweise als Stirnauflagepolster, angeordnet sein. Das Federelement kann als Elastomer-Feder (9) (siehe Fig. 5, 9,13 und 14) aus einem Elastomer wie beispielsweise Silikon oder TPE oder aus verschiedenen Elastomeren die miteinander verbunden sind oder alternativ als Druckfeder (9') aus Metall wie aus Edelstahl oder aus Kunststoff wie POM ausgeführt sein. Eine Druckfeder aus POM kann eine preiswerte Alternative zur Druckfeder aus Edelstahl sein.
Je nach Anzugskraft der Kopfbänderung drückt sich das Federelement (9, 9') innerhalb des Stützkörpers mehr oder weniger feste zusammen und drückt das Stirnauflagepolster auf die Stirn des Anwenders und bietet so immer den optimalen patientengerechten Abstand des Patienten Interfaces. Selbst wenn der Patient im Schlaf seine Lage verändert, gleicht das Federelement (9, 9') die Veränderung wieder aus.
Die Federkraft des Federelementes (9, 9') und die Kraft des Stirnauflagepolsters (8) können so ausgelegt sein, dass sich beim Anziehen der Bänderung zuerst das Stirnauflagepolster (8) der Form des Anwendergesichts- bzw. der Anwenderstirn anpasst und erst dann die Federkraft des Federelementes (9, 9') wirkt. Die Federkräfte von Federelement (9, 9') und Stirnauflagepolster (8) können auch gleich sein. Es ist ebenfalls möglich die Federkraft des Federelementes (9, 9') geringer als die Stirnauflagepolsters (8) auszuführen.

Figur 3 zeigt, dass die zylindrische Führung (10) außen Nasen (12) aufweist, die - bedingt durch die Schlitze (13) in der Führung (10) - auf Stegen (12a) angeordnet sind. Die Stege (12a) sind leicht federnd und greifen mit den Nasen (12) bei der Montage der Stützkörper in die Aufnahmebereiche (17) der ebenfalls zylindrisch ausgeführten Aufnahme der Stützkörper (2a) ein und verrasten dort. Diese verhindert Verrastung, dass die Stirnstütze ungewollt herausrutschen kann. Zur verdrehsicheren Montage befinden sich außen an der zylindrischen Federaufnahme (10) Führungselemente (11), die in entsprechenden innenliegende Führungsnuten (16) der Aufnahme (2a) greifen und dort geführt werden.

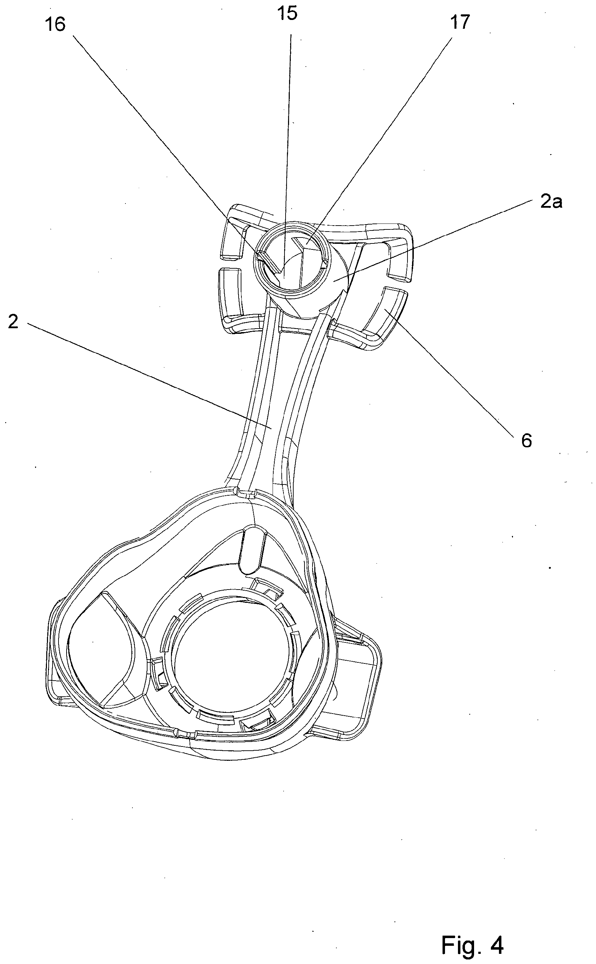

Figur 4 zeigt die zylindrische Aufnahme (2a), welche am Ende eine Anlagefläche (15) für das Federelement (9, 9') aufweist. Der Federweg (24) wird durch die Anlagefläche (15) und die gewählte Länge der Aufnahmebereiche (17) begrenzt. In einem Ausführungsbeispiel gleitet die Führung (10) teleskopierend in dem Aufnahmebereich (17), wobei das Federelement vollständig von beiden überdeckt ist.

Figur 5 zeigt das Federelement als Elastomer-Feder (9). Die Elastomer-Feder (9) erhält ihre Funktion durch die X-förmige Anordnung von Streben (9c) und die Auswahl des Materials. Bei Druck auf das Federelement (9) verändert sich der Winkel in der X-förmigen Federstruktur und das Federelement wird kürzer. Freischnitte (9d) in den X-förmigen Streben und die zwischen den X-en angeordneten Verstrebungen (9d) verhindern ein Verformen der Elastomer-Feder (9) bei Druck. Die hier dreieckigen Freischnitte (9h) geben einen Federraum frei. Grundsätzlich kann durch die Geometrie und Form solcher Freischnitte (9h) die Federeigenschaft mitbestimmt werden.

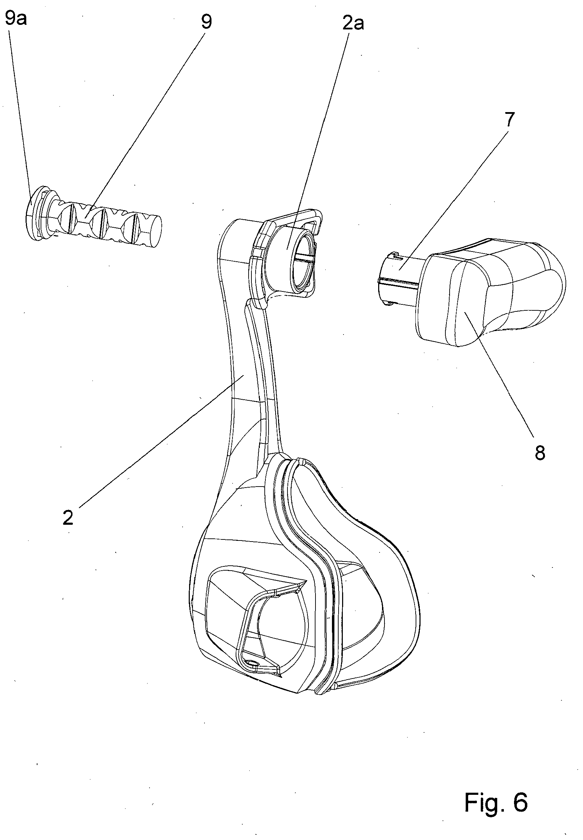

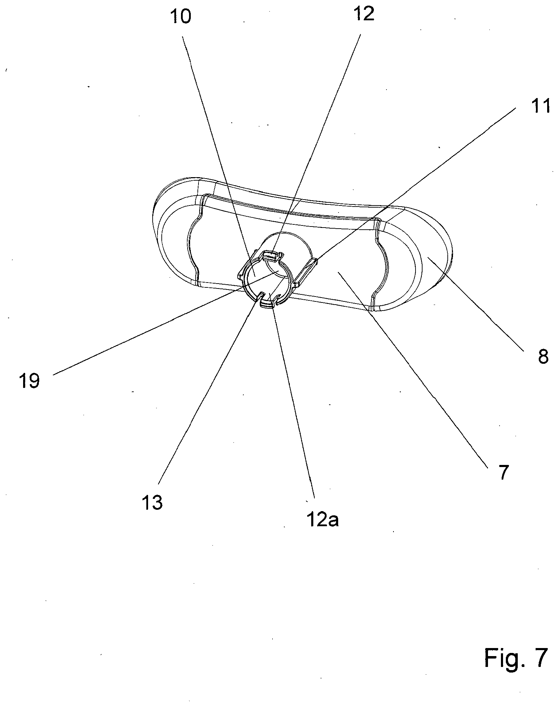

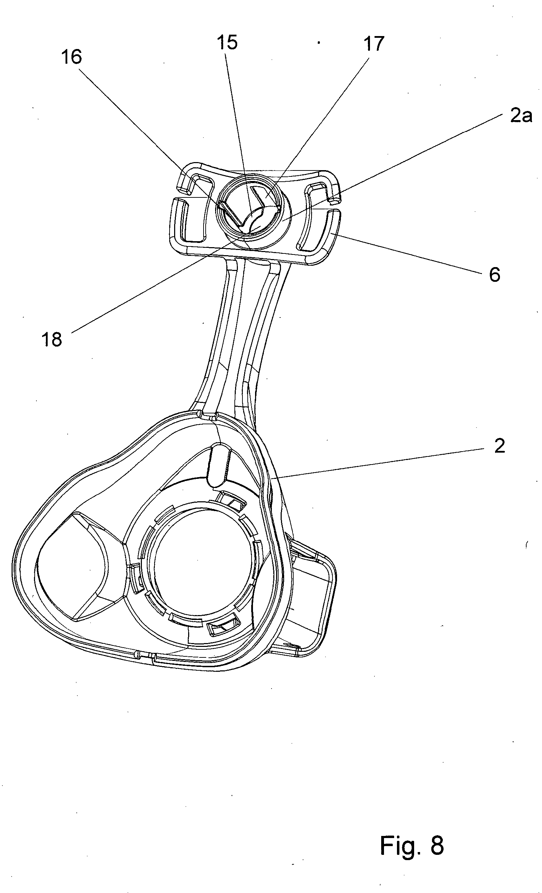

Fig. 6 zeigt den Aufbau in einer Explosionsdarstellung. Die Figuren 7 bis 9 zeigen die entsprechenden Einzelteile. Der Unterschied zur 1. Variante ist die Elastomer-Feder (9), die in dieser Ausführungsform nicht in der Öffnung (14) im Stützkörper (7) gehalten wird, sondern mit seiner Verdickung (9a) und einem Hinterschnitt (9b) in der Öffnung (18) der Stirnstützenaufnahme (2a) gehalten wird. Zur Montage wird die Elastomer-Feder (9) von vorne in die Öffnung (18) der Stirnstützenaufnahme (2a) eingeführt und so weit gezogen, bis die Fläche (15) im Hinterschnitt (9b) liegt. Danach wird die zylindrische Führung (10) der Stützkörper (7) über das Federelement geführt und verrastet ebenfalls, wie bereits in Variante 1 beschrieben, mit den Nasen (12) in den Aufnahmebereichen (17) der Stirnstützenaufnahme (2a). Die Elastomer-Feder (9) liegt in dieser Variante an der Anlagefläche (19) in dem Stützkörper (7) an. Der Federweg (24) wird durch die Anlagefläche (19) und die gewählte Länge der Aufnahmebereiche (17) begrenzt.

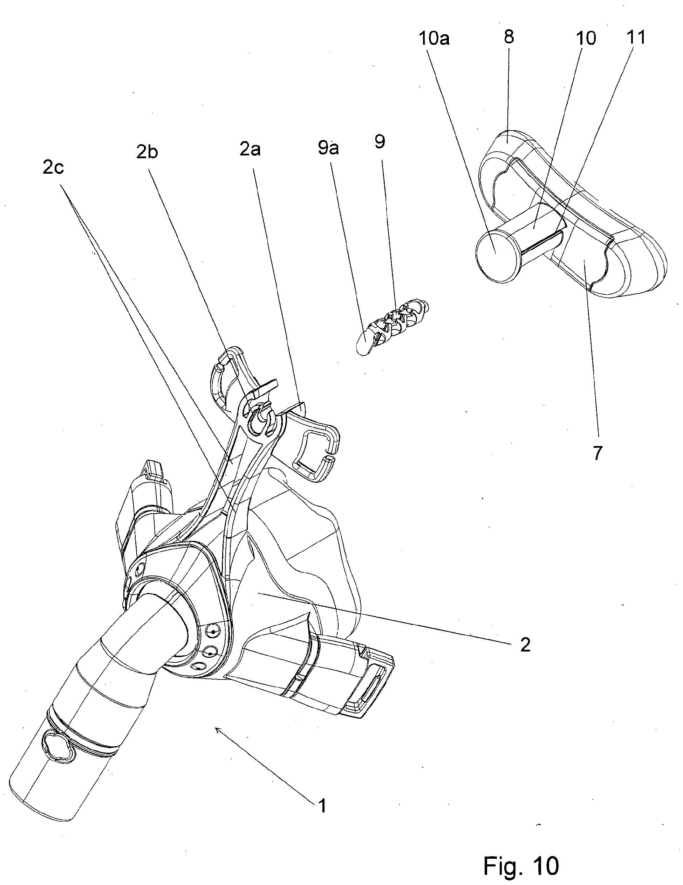

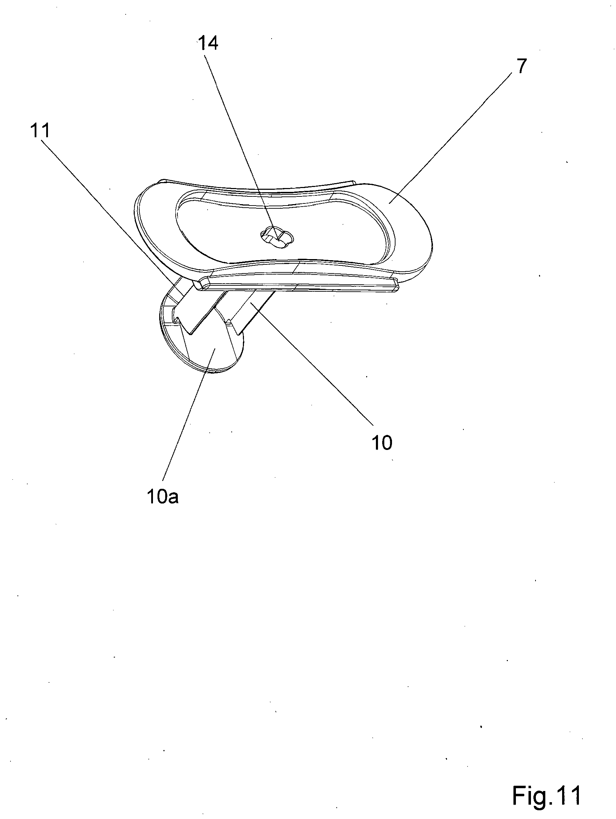

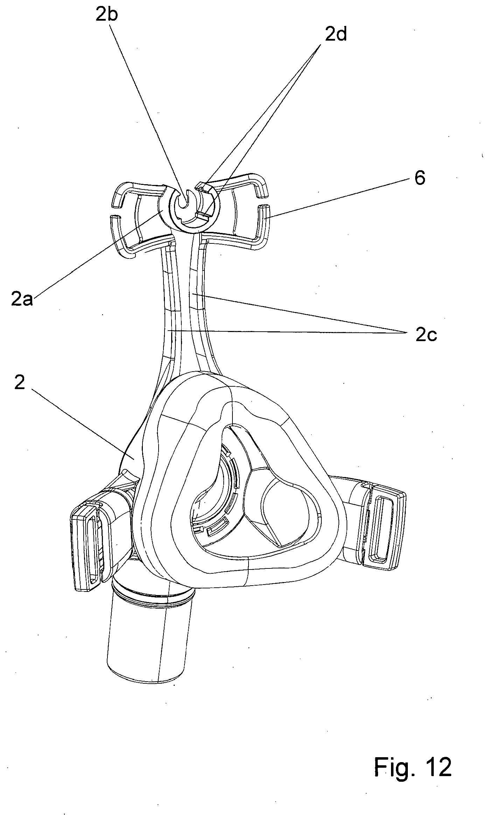

Fig. 10 zeigt eine weitere Ausführungsform zur Positionierung einer Stirnauflage eines Patienten Interfaces (1) mittels eines Federelementes (9). Die entsprechenden Einzelteile sind in den Figuren 11 bis 13 dargestellt. Zur Montage wird das Federelement (9) mittels einer Lasche (9a) und einem Hinterschnitt (9b) in den Aufnahmebereich (2b) der Aufnahme (2a) eingeknüpft. Das Federelement (9) weist an dem entgegengesetzten Ende ein Zentrierelement (9f) auf, das in die Öffnung (14) in der Anlagefläche (19) des Stützkörpers (7) eingeführt wird und ein seitliches Bewegen bzw. Verrutschen des Federelementes (9) in dem Stützkörper verhindert. Die Verbindungsstege (2c), die zwischen Maskenkörper (2) und Aufnahmebereich der Stirnstütze (2a) sind so gestaltet, dass sie vorzugsweise ein Zusammendrücken möglich machen und dadurch den Aufnahmebereich (2a) aufweiten und die Montage des Stützkörpers (7) ermöglichen.
Zur Montage werden die die beiden Verbindungsstege (2c) zusammengedrückt und der Stützkörper in die Öffnung der Aufnahme (2a) geklickt. Dabei muss das Zentrierelement (9f) des Federelementes (9) in der Öffnung (14) richtig positioniert sein. Eine Verdreh-Sicherung des Stützkörpers (7) wird durch Führungselemente in Form eines Absatzes (11) am Umfang der Federelementaufnahme (10) des Stützkörpers (7) und Fasen (2d) in der Aufnahme (2a) gewährleistet.
Die Lasche (10a) an dem Stützkörper (7), die im montierten Zustand vor der Aufnahme der Stützkörper (2a.) liegt, bildet dort einen Anschlag in Richtung zum Patienten. Der Verstellweg des Stützkörpers wird durch die Anlage des Federelementes (9) in dem Stützkörper (7) und die Länge der Federelementaufnahme (10) an dem Stützkörper begrenzt. Der minimale Verstellpunkt des Stützkörpers (7) zur Stirn des Patienten ist erreicht, wenn die Lasche (10a) vorne an der Aufnahme des Stützkörpers (2a) anliegt. Der maximale Verstellpunkt des Stützkörpers (7) ist erreicht, wenn die Aufnahme (2a) an dem Stützkörper (7) anliegt.

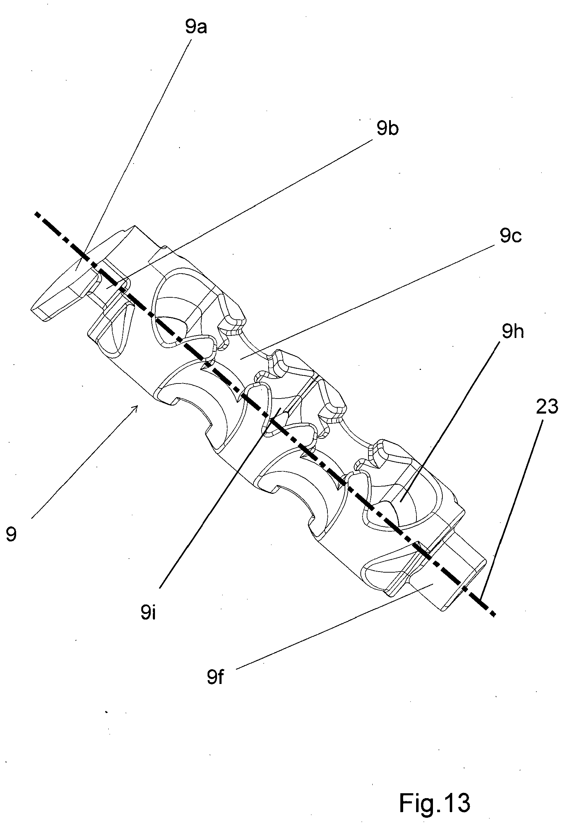

Figur 13 zeigt das Federelement als Elastomer-Feder (9). Die - Elastomer-Feder (9) hat eine Achse (23) in ihrer Längsrichtung. Eine Kraftbeaufschlagung erfolgt immer in Richtung der Achse (23) Die Elastomer-Feder (9) erhält ihre Funktion durch die Anordnung von Streben (9c) und Freischnitten (9h, 9i). Bei Druck auf das Federelement (9) verändert sich der Winkel in den Freischnitten (9h, 9i) und das Federelement wird kürzer. Die hier unterschiedlichen Freischnitte (9h, 9i) bilden jeweils einen Federraum. Hier ist Geometrie so gewählt, dass der Freischnitt (9i) weicher ist und somit bei Krafteinwirkung auf das Federelement zuerst einfedert. Der Freischnitt (9h) ist aufgrund seiner von Freischnitt (9i) abweichenden Geometrie härter und federt daher erst nach dem Freischnitt (9i) voll ein. Grundsätzlich kann durch die Geometrie und Form solcher Freischnitte (9h, 9i) die Federeigenschaft mitbestimmt werden.

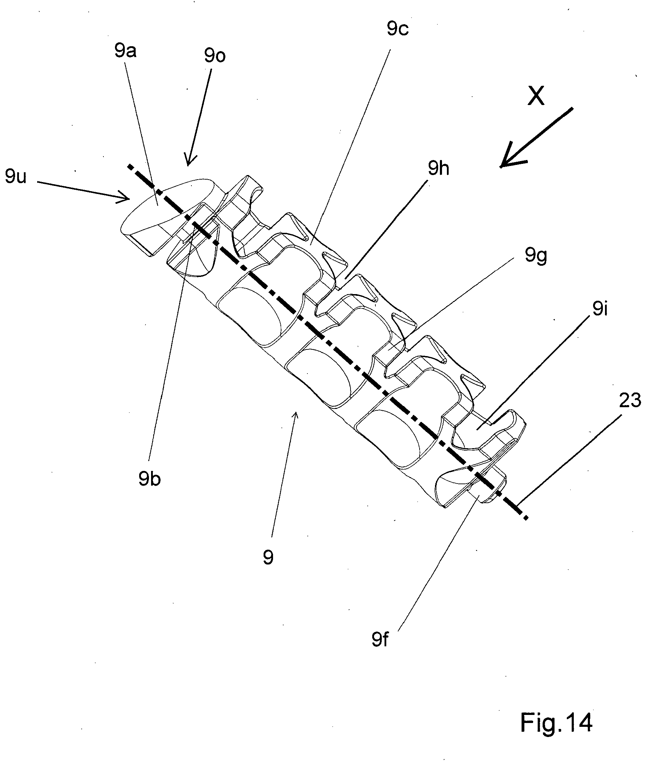

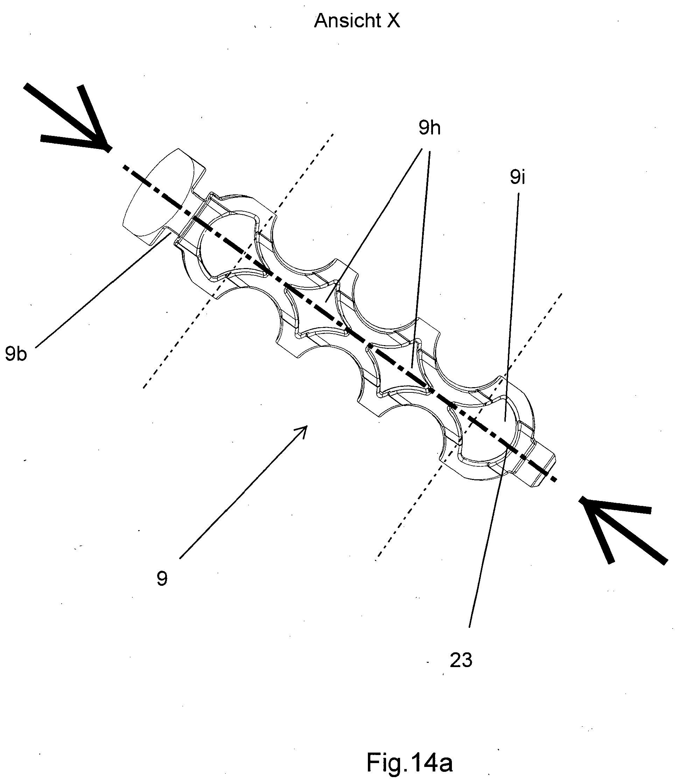

Figur 14 und Fig 14a zeigen das Federelement welches senkrecht zur Achse (23) eine ersten Teilbereich (9h), hier durch den Freischnitt als viereckige geometrische Aussparung, mit einer ersten Eigenschaft, die hier als höhere Federkonstante oder als härterer Abschnitt ausgebildet ist. Ortsfern zum ersten Teilbereich ist ein zweiter Teilbereich (9i), hier durch den Freischnitt als dreieckige geometrische Aussparung (mit gerundeter Basis), ausgebildet, der eine zweiten Eigenschaft aufweist. Die zweite Eigenschaft ist hier als geringere Federkonstante, als im ersten Teilbereich, oder als weicherer Abschnitt des Federelementes ausgebildet.
Figur 14 zeigt das Federelement in einer seitlichen Ansicht mit der Achse (23) die das Federelement in eine obere (9o) und eine untere (9u) Hälfte unterteilen. Die obere (9o) und die untere (9u) Hälfte des Federelementes stellen hier einen ersten Teilbereich und einen zweiter Teilbereich dar. Bezogen auf die Achse ist die obere Hälfte anders gestaltet als die untere Hälfte. Das Federelement ist somit nicht symmetrisch aufgebaut. Das Federelement weist senkrecht zur Achse (23) einen ersten Teilbereich (9o) mit einer ersten Eigenschaft und senkrecht zum ersten Teilbereich und zur Achse (23) einen zweiten Teilbereich (9u) mit einer zweiten Eigenschaft auf. Vorliegend sind im ersten Teilbereich (9o) erhöhte x-förmige Strukturen zu erkennen, die das Federelement hier relativ steifer machen als im zweiten Teilbereich (9u). Im zweiten Teilbereich (9u) finden sich keine Versteifungen oder Erhöhungen, weshalb sich das Federelement bei Kraftbeaufschlagung in Richtung zur Achse (23) nach unten hin zum zweiten Teilbereich (9u) durchbiegt. Dies wird genutzt bei der Einbaulage des Federelementes in die Aufnahme (2a, 10). In der Aufnahme ist der zweite Teilbereich (9u) so angeordnet, dass der sich an der Wandung der Aufnahme des Federelementes abstützen kann. Der erste Teilbereich (9o) kann somit von außen zugänglich und ohne Führung durch die Aufnahme des Federelementes gestaltet werden. Dies bietet Vorteile bei der Reinigung des PI.
Betrachtet man zusätzlich die Teilbereiche (9h, 9i), so weist das Federelement zumindest zwei Teilbereiche auf. Vorliegend weist das Federelement zumindest vier Teilbereiche (9h, 9i, 9o, 9u) auf.

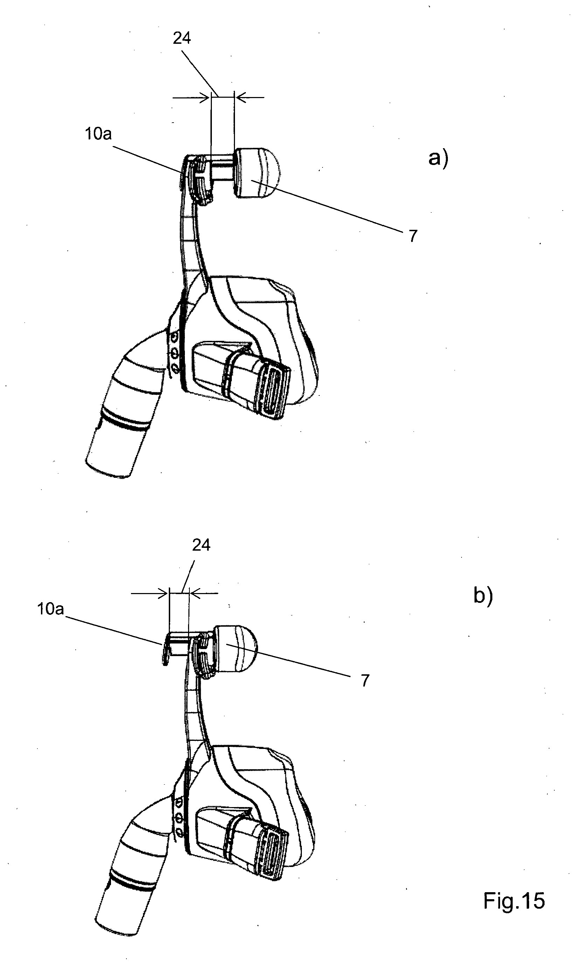

Figur 15a und 15b zeigen den Verstellweg oder Federweg (24) des Stützkörpers (7) der durch den Anschlag (10a) begrenzt ist.
Für alle Ausführungsformen der Erfindung kann gelten: Das Federelement welches senkrecht zur Achse (23) eine ersten Teilbereich (9h), hier durch den Freischnitt als viereckige geometrische Aussparung, mit einer ersten Eigenschaft, die hier als höhere Federkonstante oder als härterer Abschnitt ausgebildet ist. Ortsfern zum ersten Teilbereich ist ein zweiter Teilbereich (9i), hier durch den Freischnitt als dreieckige geometrische Aussparung (mit gerundeter Basis), ausgebildet, der eine zweiten Eigenschaft aufweist. Die zweite Eigenschaft ist hier als geringere Federkonstante, als im ersten Teilbereich, oder als weicherer Abschnitt des Federelementes ausgebildet.
Der erste und zweite Teilbereich des Federelemtes können auch einander gegenüberliegen wobei der erste Teilbereich eine höhere Steifigkeit oder Federkonstante aufweist als der zweite Teilbereich.

Ein Teilbereich kann durch geometrische Strukturen (wie beispielsweise Verrippung, Kontur, offene Kontur und geschlossene Kontur) eine höhere Steifigkeit oder Federkonstante aufweisen.

Ein Teilbereich kann durch unterschiedliche Materialien (beispielsweise im 2K-Verfahren verbundene unterschiedliche Shorehärten, im Bereich Shore A 20 - 80) eine erhöhte Steifigkeit oder Federkonstante aufweisen.

Ein Teilbereich kann durch unterschiedliche Wandstärken eine höhere Steifigkeit oder Federkonstante aufweisen.

Für alle Ausführungsformen der Erfindung kann auch gelten: Die Rückstellkraft des Federelementes (9, 9') und die Rückstellkraft des Stirnauflagepolsters (8) sind so ausgelegt, dass sich beim Anlegen/Anziehen der Bänderung des Patienten Interfaces (1) zuerst das Stirnauflagepolster (8) der Form des Anwendergesichts bzw. der Anwenderstirn anpasst und erst dann mit steigender Anzugkraft der Bänderung das Federelement (9, 9') sukzessiv komprimiert wird. Der Anwender bestimmt so über die Anzugkraft der Bänderung, die er selbst einstellt und somit bestimmt, die Lage des Patienteninterface relativ zur Stirn und somit die Neigung des Patienteninterface auf dem Gesicht. Erfindungsgemäß ist es auch möglich die Rückstellkraft des Federelementes (9, 9') und die Rückstellkraft des Stirnauflagepolsters (8) etwa gleich auszulegen oder die des Rückstellkraft des Stirnauflagepolsters (8) so auszulegen, dass zunächst das Federelement komprimiert wird. Erfindungsgemäß ist auch vorgesehen, dass das Federelement (8, 9, 9') durch das Stirnauflagepolster gebildet wird. Erfindungsgemäß ist auch vorgesehen, dass das Federelement (8, 9, 9') unterschiedliche Federeigenschaften aufweist.

Um dies zu gewährleisten sind vorteilhaft keine Raststufen vorgesehen. Der besondere Vorteil für den Benutzer ist die, aus dem automatischen Verstellen der Stirnstütze, resultierende stufenlose Feineinstellung. Es kann jedoch auch zumindest eine Raststufe vorgesehen werden, die jedoch durch zusätzlichen Krafteintrag überwunden werden kann.

Durch das Kräftegleichgewicht zwischen der Anzugkraft der Kopfbänderung und der Rückstellkraft des Federelementes der Stirnstütze ist jederzeit eine optimale und fehlerfreie Anpassung gewährleistet, auch wenn der Patient im Schlaf seine Lage verändert.

Überraschenderweise wurde im Rahmen der vorliegenden Erfindung festgestellt, dass bei einem Patienteninterface der erfindungsgemäßen Art ein eng definierter Verstellbereich, der im Wesentlichen dem Federweg entspricht, für den Stützkörper ausreichend ist, um etwa 90 % der Patientengesichter abzudecken. Der Federweg (24) des Federelementes (8, 9, 9') liegt daher bei allen Varianten zwischen 5 mm und 30 mm, 7 mm und 17 mm, bevorzugt bei 8 mm bis 15 mm, besonders bevorzugt bei 9 mm bis 14 mm. Der Federweg des Federelementes (9, 9') kann auch bei 12 mm liegen.
Überraschenderweise wurde im Rahmen der vorliegenden Erfindung festgestellt, dass bei einem Patienteninterface der erfindungsgemäßen Art eine eng definierte Federkonstante des Federelementes ausreichend ist, um etwa 90 % der Patienten eine passgenaue und angenehme Abstützung zu bieten.
Die Federkonstante des Federelementes (8, 9, 9') liegt im Bereich zwischen 0,1 - 2,0 N/mm, bevorzugt bei 0,1 - 1,0 N/mm oder auch bei 0,1 - 0,5 N/mm auch ein Bereich bei 0,15- 0,3 N/mm ist angedacht.

### Positionsnummern

- 1: Patient Interface
- 2: Maskenkörper
- 2a: Aufnahme der Stützkörper
- 2b: Aufnahmebereich für Feder
- 2c: Verbindungsstege

- 2d: Fasen
- 3: Maskenwulst
- 4: Schlauchkupplung
- 4a: Drehhülse
- 5: Aufnahmevorrichtung für Bänderung am Maskenkörper
- 6: Bänderungs-Aufnahme Stirnstützenträger
- 7: Stützkörper
- 8: Stirnauflagepolster
- 9: Federelement
- 9a: Verdickung, Lasche an Federelement
- 9b: Hinterschnitt an Federelement
- 9c: X-förmige Federstruktur
- 9d: Freischnitt
- 9e: Streben zwischen den X-Formen
- 9f: Zentrierelement
- 9g: Verstärkung der X-förmigen Federstruktur
- 9h, 9i: Teilbereiche des Federelementes
- 9o, 9u: Teilbereiche des Federelementes
- 9': Federelement, Druckfeder
- 10: Zylindrische Führung / Federaufnahme
- 10a: Anschlaglasche
- 11: Führungselement
- 12: Nasen
- 12a: Stege
- 13: Schlitze
- 14: Öffnung für Federelement
- 15: Anlagefläche für Federelement in Aufnahme (2a)
- 16: Führungsnuten
- 17: Aufnahmebereiche
- 18: Öffnung für Federelement in Aufnahme (2a)
- 19: Anlagefläche für Federelement in Stützkörper (7)
- 20: Bänderung
- 21: Klettverbindung
- 22: Bänderungsenden
- 23: Achse
- 24: Federweg

## Patentansprüche

1. Verstellvorrichtung für die Stirnstütze eines Patienten Interface (1), welches mittels einer Kopfbänderung (20) am Kopf des Patienten fixiert wird, mit einem Stützkörper (7) und zumindest einem Federelement (9, 9') wobei das Patienten Interface (1) zumindest eine Bänderungs-Aufnahme (5, 6) aufweist und die Anzugskraft der Kopfbänderung (20) über die Bänderungs-Aufnahme (5, 6) auf das Federelement (9, 9') übertragen wird, sodass das Federelement (9, 9') komprimiert wird **dadurch gekennzeichnet, dass** das Federelement (9, 9') einen Federweg (24) im Bereich 5 mm bis 30 mm bereitstellt und wobei die Rückstellkraft des Federelementes (9, 9') und die Rückstellkraft des Stirnauflagepolsters (8) so ausgelegt sind, dass sich beim Anlegen/Anziehen der Bänderung des Patienten Interfaces (1) zuerst das Stirnauflagepolster (8) der Form des Anwendergesichts bzw. der Anwenderstirn anpasst und erst dann mit steigender Anzugkraft der Bänderung das Federelement (9, 9') sukzessiv komprimiert wird und der Anwender so über die Anzugkraft der Bänderung, die Lage des Patienteninterface relativ zur Stirn und somit die Neigung des Patienteninterface auf dem Gesicht bestimmt, wobei keine Raststufen für das Federelement vorgesehen sind, woraus eine stufenlose Feineinstellung der Stirnstütze resultiert.

2. Vorrichtung nach Anspruch 1 **dadurch gekennzeichnet, dass** die Kennlinie des Federelementes annähernd linear ist.

3. Vorrichtung nach zumindest einem der vorhergehenden Ansprüche **dadurch gekennzeichnet, dass** die Kennlinie des Federelementes durch Verwendung unterschiedlicher Elastomermaterialien und/oder unterschiedlicher Geometrien und/oder geometrischer Aussparungen innerhalb des Federelementes mehrstufig ist.

4. Vorrichtung nach zumindest einem der vorhergehenden Ansprüche **dadurch gekennzeichnet, dass** Federelement eine Achse (23) aufweist in deren Richtung die Feder gestaucht wird und die Achse (23) im Wesentlichen senkrecht zur Stirn des Anwenders orientiert ist.

5. Vorrichtung nach Anspruch 4 **dadurch gekennzeichnet, dass** das Federelement senkrecht zur Achse (23) einen ersten Teilbereich mit einer ersten Federkonstante und senkrecht zum ersten Teilbereich und zur Achse (23) einen zweiten Teilbereich mit einer zweiten Federkonstante aufweist.

6. Vorrichtung nach zumindest einem der vorhergehenden Ansprüche **dadurch gekennzeichnet, dass** die erste Federkonstante ungleich der zweiten Federkonstante ist.

7. Vorrichtung nach zumindest einem der vorhergehenden Ansprüche **dadurch gekennzeichnet, dass** das Federelement (9, 9') einen ersten und einen zweiten Teilbereich aufweist, die einander gegenüberliegen wobei der erste Teilbereich eine höhere Steifigkeit aufweist als der zweite Teilbereich.

8. Vorrichtung nach Anspruch 7 **dadurch gekennzeichnet, dass** ein Teilbereich durch geometrische Strukturen und/oder unterschiedliche Materialien und/oder unterschiedliche Wandstärken eine höhere Steifigkeit aufweist.

9. Verstellvorrichtung für ein Patienten Interface (1) nach einem der vorhergehenden Ansprüche **dadurch gekennzeichnet, dass** das Federelement eine Achse (23) aufweist, in deren Richtung das Federelement gestaucht wird, wobei das Federelement senkrecht zur Achse (23) einen ersten Teilbereich mit einer ersten Federkonstante und ortsverschieden zum ersten Teilbereich einen zweiten Teilbereich mit einer zweiten Federkonstante aufweist.

10. Vorrichtung nach zumindest einem der vorhergehenden Ansprüche **dadurch gekennzeichnet, dass** das Federelement (9) X-förmig angeordnete Streben (9c) aufweist.

11. Vorrichtung nach zumindest einem der vorhergehenden Ansprüche **dadurch gekennzeichnet, dass** der Stützkörper (7) zumindest teilweise in eine horizontale Aufnahme (2a) des Maskenkörpers (2) eingreift und das Federelement (9, 9') im montierten Zustand teilweise in die Aufnahme (2a) und teilweise in eine Führung (10) eintaucht.

12. Vorrichtung nach Anspruch 11 **dadurch gekennzeichnet, dass** die Führung (10) Nasen (12) aufweist und der Stützkörper (7) in den Aufnahmebereich (17) der horizontalen Aufnahme (2a) greift und dort verrastet.

## Claims

1. An adjusting device for the forehead support of a patient interface (1) which is fixed by means of a head harness (20) to the head of the patient, comprising a support body (7) and at least one spring element (9, 9'), wherein the patient interface 1) has at least one harness receiver (5, 6) and the tightening force of the head harness (20) is transmitted via the harness receiver (5, 6) to the spring element (9, 9') such that the spring element (9, 9') is compressed, **characterised in that** the spring element (9, 9') provides a spring excursion (24) ranging from 5 mm to 30 mm, and wherein the restoring force of the spring element (9, 9') and the restoring force of the forehead supporting pad (8) are designed such that when the harness of the patient interface (1) is applied/tightened, the forehead supporting pad (8) is initially adapted to the shape of the face of a user and/or the forehead of a user and only then with the increasing tightening force of the harness is the spring element (9, 9') successively compressed and thus, via the tightening force of the harness, the user defines the position of the patient interface relative to the forehead and thus the inclination of the patient interface on the face, wherein no latching steps are provided for the spring element, resulting in a stepless fine adjustment of the forehead support.

2. The device according to Claim 1, **characterised in that** the characteristic curve of the spring element is approximately linear.

3. The device according to at least one of the preceding claims, **characterised in that** the characteristic curve of the spring element has multiple stages, due to the use of different elastomer materials and/or different geometries and/or geometric recesses inside the spring element.

4. The device according to at least one of the preceding claims, **characterised in that** the spring element has an axis (23), the spring being compressed in the direction thereof, and the axis (23) is oriented substantially perpendicular to the forehead of the user.

5. The device according to Claim 4, **characterised in that** perpendicular to the axis (23) the spring element has a first partial region with a first spring constant and, perpendicular to the first partial region and to the axis (23), the spring element has a second partial region with a second spring constant.

6. The device according to at least one of the preceding claims, **characterised in that** the first spring constant is different from the second spring constant.

7. The device according to at least one of the preceding claims, **characterised in that** the spring element (9, 9') has a first and a second partial region which oppose one another, wherein the first partial region has a greater rigidity than the second partial region.

8. The device according to Claim 7, **characterised in that** a partial region has a greater rigidity due to the geometric structures and/or different materials and/or different wall thicknesses.

9. An adjusting device for a patient interface (1) according to one of the preceding claims, **characterised in that** the spring element has an axis (23); the spring element being compressed in the direction thereof, wherein perpendicular to the axis (23) the spring element has a first partial region with a first spring constant and, at a different location from the first partial region, the spring element has a second partial region with a second spring constant.

10. The device according to at least one of the preceding claims, **characterised in that** the spring element (9) has struts (9c) arranged in an X-shaped manner.

11. The device according to at least one of the preceding claims, **characterised in that** the support body (7) engages at least partially in a horizontal receiver (2a) of the mask body (2) and in the mounted state the spring element (9, 9') is immersed partially in the receiver (2a) and partially in a guide (10).

12. The device according to Claim 11, **characterised in that** the guide (10) has lugs (12) and the support body (7) grips in the receiving region (17) of the horizontal receiver (2a) and is latched therein.

## Revendications

1. Dispositif de réglage de l'appui-front d'une interface patient (1), laquelle est fixée sur la tête du patient au moyen d'un serre-tête (20), avec un corps d'appui (7) et au moins un élément à ressort (9, 9'), dans lequel l'interface patient (1) présente au moins un logement de bandeau (5, 6) et la force de serrage du serre-tête (20) est transmise à l'élément à ressort (9, 9') par le biais du logement de bandeau (5, 6), de sorte que l'élément à ressort (9, 9') est comprimé, **caractérisé en ce que** l'élément à ressort (9, 9') assure une course de ressort (24) dans la plage de 5 mm à 30 mm et dans lequel la force de rappel de l'élément à ressort (9, 9') et la force de rappel du rembourrage d'appui frontal (8) sont conçues de manière à ce que le rembourrage d'appui frontal (8) épouse d'abord la forme du visage et du front de l'utilisateur lors de la pose / du serrage du bandeau de l'interface patient (1), que seulement ensuite l'élément à ressort (9, 9') soit comprimé successivement à mesure que la force de serrage du bandeau augmente et que l'utilisateur détermine de cette façon la position de l'interface patient par rapport au front et ainsi l'inclinaison de l'interface patient sur le visage par le biais de la force de serrage du bandeau, dans lequel il n'est prévu aucun cran d'arrêt de l'élément à ressort, d'où l'ajustement de précision continu de l'appui-front.

2. Dispositif selon la revendication 1 **caractérisé en ce que** la, courbe caractéristique de l'élément à ressort est presque linéaire.

3. Dispositif selon l'une au moins des revendications précédentes, **caractérisé en ce que** la courbe caractéristique de l'élément à ressort décrit plusieurs paliers moyennant l'emploi de matériaux élastomères différents et / ou de géométries différentes et / ou d'évidements géométriques à l'intérieur de l'élément à ressort.

4. Dispositif selon l'une au moins des revendications précédentes, **caractérisé en ce que** l'élément à ressort présente un axe (23), dans la direction duquel le ressort est écrasé, et l'axe (23) est orienté sensiblement à la perpendiculaire du front de l'utilisateur.

5. Dispositif selon la revendication 4 **caractérisé en ce que** l'élément à ressort présente une première zone partielle avec une première constante de rappel *(raideur)* perpendiculairement à l'axe (23) et une seconde zone partielle avec une seconde constante de rappel perpendiculairement à la première zone partielle et à l'axe (23).

6. Dispositif selon l'une au moins des revendications précédentes, **caractérisé en ce que** la première constante de rappel est différente de la seconde constante de rappel.

7. Dispositif selon l'une au moins des revendications précédentes, **caractérisé en ce que** l'élément à ressort (9, 9') présente une première et une seconde zone partielle, qui sont en vis-à-vis l'une de l'autre, dans lequel la première zone partielle présente une raideur plus élevée que la seconde zone partielle.

8. Dispositif selon la revendication 7 **caractérisé en ce qu'**une zone partielle présente une raideur supérieure moyennant des structures géométriques et / ou des matériaux différents et / ou des épaisseurs de paroi différentes.

9. Dispositif de réglage d'une interface patient (1) selon l'une des revendications précédentes, **caractérisé en ce que** l'élément à ressort présente un axe (23), dans la direction duquel le ressort est écrasé, dans lequel l'élément à ressort présente une première zone partielle avec une première constante de rappel à la perpendiculaire de l'axe (23) et une seconde zone partielle avec une seconde constante de rappel en divers endroits par rapport à la première zone partielle.

10. Dispositif selon l'une au moins des revendications précédentes, **caractérisé en ce que** l'élément à ressort (9) présente des croisillons (9c) disposés en forme de X.

11. Dispositif selon l'une au moins des revendications précédentes, **caractérisé en ce que** le corps d'appui (7) s'engage au moins en partie dans un logement horizontal (2a) de la coque de masque (2) et l'élément à ressort (9, 9') s'enfonce à l'état monté en partie dans le logement (2a) et en partie dans une douille de guidage (10).

12. Dispositif selon la revendication 11 **caractérisé en ce que** la douille de guidage (10) présente des ergots (12) et le corps d'appui (7) s'engage dans la zone de réception (17) du logement horizontal (2a) et s'y enclenche.
